(19) Europäisches Patentamt
European Patent Office
Office européen des brevets

(11) Veröffentlichungsnummer: **0 012 925**
**B1**

(12) **EUROPÄISCHE PATENTSCHRIFT**

(45) Veröffentlichungstag der Patentschrift: **10.11.82**

(51) Int. Cl.³: **C 07 D 215/56,** A 61 K 31/47

(21) Anmeldenummer: **79105088.3**

(22) Anmeldetag: **11.12.79**

(54) **Neue Chinoloncarbonsäure-Derivate, Verfahren zu ihrer Herstellung und diese Verbindungen enthaltende pharmazeutische Präparate.**

(30) Priorität: **28.12.78 DE 2856908**

(43) Veröffentlichungstag der Anmeldung:
**09.07.80 Patentblatt 80/14**

(45) Bekanntmachung des Hinweises auf die Patenterteilung:
**10.11.82 Patentblatt 82/45**

(84) Benannte Vertragsstaaten:
**AT BE CH DE FR GB IT LU NL SE**

(56) Entgegenhaltungen:
**GB-A-1 120 870**

(73) Patentinhaber: **SCHERING AKTIENGESELLSCHAFT Berlin und Bergkamen,**
**Müllerstrasse 170/178 Postfach 65 03 11,**
**D-1000 Berlin 65 (DE)**

(72) Erfinder: **Albrecht, Rudolf, Dr., Wunsiedeler Weg 37,**
**D-1000 Berlin 46 (DE)**
Erfinder: **Schröder, Eberhard, Dr.,**
**Bergengruenstrasse 44 a, D-1000 Berlin 38 (DE)**
Erfinder: **Böttcher, Irmgard, Dr., Rodelbahnpfad 5,**
**D-1000 Berlin 28 (DE)**

**EP 0 012 925 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Neue Chinoloncarbonsäure-Derivate, Verfahren zu ihrer Herstellung und diese Verbindungen enthaltende pharmazeutische Präparate

Die Erfindung betrifft den in den Patentansprüchen gekennzeichneten Gegenstand.

Es ist bekannt, dass pharmazeutische Präparate, die als Wirkstoff das Natriumsalz der Cromoglicinsäure enthalten, nach Inhalation die Freisetzung der eine Bronchokonstriktion auslösenden Mediatoren, wie Histamin und SRS-A aus den Mastzellen der Lunge inhibieren und sich somit bei prophylaktischer Anwendung sehr gut zur Behandlung des allergischen Asthmas eignen. (J. Med. Chem., 15, 1972, 583).

Die in den Patentansprüchen 1 bis 14 gekennzeichneten Chinoloncarbonsäure-Derivate besitzen die gleiche Eigenschaft wie das Natriumsalz der Cromoglicinsäure, haben aber gegenüber jener Verbindung den Vorzug, dass sie darüberhinaus auch eine für die Asthmatherapie günstige entzündungshemmende und antibacterielle Wirksamkeit zeigen.

Ferner konnten die aus der GB-A-1 120 870 bekannten veterinärmedizinischen Chinolincarbonsäure-Derivate die erfindungsgemässen Verbindungen ebenfalls nicht nahelegen.

Die gegenüber den vorstehend genannten Verbindungen unterschiedlich strukturierten Chinoloncarbonsäure-Derivate der allgemeinen Formel I gemäss Anspruch 1 können als Substituenten $R_1$ eine 1 bis 6 Kohlenstoffatome oder insbesondere eine 1 bis 4 Kohlenstoffatome enthaltende Alkylgruppe, wie zum Beispiel die Methylgruppe, die Äthylgruppe, die Propylgruppe, die Isopropylgruppe, die Butylgruppe, die Isobutylgruppe, die tert.-Butylgruppe, die Pentylgruppe oder Hexylgruppe tragen.

Als Substituenten $R_2$ können die Chinoloncarbonsäure-Derivate eine 1 bis 8 Kohlenstoffatome oder insbesondere eine 1 bis 5 Kohlenstoffatome enthaltende Acylgruppe, vorzugsweise eine Alkynoylgruppe oder eine Benzoylgruppe besitzen. Geeignete Alkanoylgruppen sind beispielsweise die Formylgruppe, die Acetylgruppe, die Propionylgruppe, die Butyrylgruppe oder die Oktanoylgruppe.

Zwecks Erhöhung ihrer Wasserlöslichkeit können die Chinoloncarbonsäure-Derivate der allgemeinen Formel I mit X in der Bedeutung eines Wasserstoffatomes mit physiologisch unbedenklichen Basen in ihre Salze überführt werden. Geeignete Salze sind beispielsweise die Alkalimetallsalze (vorzugsweise Lithiumsalze, Natriumsalze oder Kaliumsalze), die Erdalkalimetallsalze (vorzugsweise Magnesiumsalze oder Calziumsalze), Kupfer(II)-salze, Ammoniumsalze oder Salze organischer Amine (beispielsweise Triäthanolamin, Piperazin, N-Methylglycamine usw.).

Es wurde bereits erwähnt, dass die Chinoloncarbonsäure-Derivate der allgemeinen Formel I gemäss Anspruch 1 sich durch eine antiallergische, entzündungshemmende und antibacterielle Wirksamkeit auszeichnen.

Zur Bestimmung der antiallergischen Wirksamkeit kann man die Hemmung der Mediatorenfreisetzung am Modell der passiven cutanen Anaphylaxie der Ratte (PCA) messen (Ovary, Z: Immunological Methods, ed. J.F. Ackroyd, Blackwell Scientific Publ. Oxford 1964, p. 259). Dazu werden Ratten durch intracutane Injektion verschiedener Verdünnungen (1 : 8 und 1 : 16) eines standardisierten IgE-haltigen Hyperimmunserums passiv sensibilisiert. Nach 48 Stunden erfolgt die intravenöse Applikation der zu prüfenden Verbindung, gelöst als Natriumsalz in physiologischer Kochsalzlösung, und von Ovalbumin als Allergen, kombiniert mit einem Farbstoff zur besseren Messbarkeit der lokalen allergischen Reaktion.

Es wird die Hemmung der allergischen Reaktion in Gegenwart der Prüfverbindung im Vergleich zu der allergischen Reaktion ohne inhibierendes Agens gemessen. Die erhaltenen Werte sind in Tabelle 1 angegeben. Wie ersichtlich, liegt eine Gleichwertigkeit zwischen den erfindungsgemässen Verbindungen und Cromoglicinsäure vor.

Die entzündungshemmende Wirkung wird im Carrageenin-Test gemessen (Winter, C.A., E.A. Risley and G.W. Nuss, Proc. Soc. exp. Biol. Med. 111, 544–547, 1962). Die Ergebnisse sind in Tabelle 2 zusammengestellt. Wie ersichtlich, zeigen die erfindungsgemässen Verbindungen eine deutliche entzündungshemmende Wirksamkeit, wogegen die Cromoglicinsäure nur eine äusserst schwache oder gar keine entsprechende Wirkung aufweist.

Die antibakterielle Wirkung wird in einem Reihenverdünnungstest in vitro gemessen. Die Ergebnisse sind in Tabelle 3 zusammengestellt. Wie ersichtlich, zeigen die erfindungsgemässen Verbindungen eine antibakterielle Wirkung gegen Diplococcus pneumoniae und eine Reihe von Stämmen von Staphylococcus aureus, während Cromoglicinsäure gegen alle untersuchten Bakterien unwirksam ist.

Tabelle 1
Antiallergische Wirkung, dargestellt im PCA-Test der Ratte bei i.v.-Applikation

| Verbindung | Dosis mg/kg | % Hemmung IgE 1:8 | IgE 1:16 |
|---|---|---|---|
| 1,3-Bis-(1-äthyl-3-carboxy-1,4-dihydro-4-oxo-7-chinolyloxy)-2-propanol | 10 | 84 | 90 |
| | 5 | 59 | 69 |
| 1,3-Bis-(1-butyl-3-carboxy-1,4-dihydro-4-oxo-7-chinolyloxy)-2-propanol | 10 | 71 | 85 |
| 1,3-Bis-(3-carboxy-1,4-dihydro-1-methyl-4-oxo-7-chinolyloxy)-2-propanol | 5 | 62 | 69 |
| Cromoglicinsäure | 10 | 60 | 84 |
| | 5 | 63 | 67 |

Tabelle 2
Entzündungshemmende Wirkung im Carrageenin-Test bei i.v.- und p.o.-Applikation

| Verbindung | Dosis mg/kg | Applikationsart | % Hemmung |
|---|---|---|---|
| 1,3-Bis-(1-äthoxy-3-carboxy-1,4-dihydro-4-oxo-7-chinolyloxy)- | 10 | i.v. | 44 |
| 2-propanol | 75 | p.o. | 28 |
| Cromoglicinsäure | 10 | i.v. | 10 |
| | 75 | p.o. | 0 |

Tabelle 3
Antibakterielle Wirkung in vitro
Minimale Hemmkonzentration in µg/ml gegen

| gegen | 1,3-Bis-(1-äthyl-3-carboxy-1,4-dihydro-4-oxo-7-chinolyloxy)-2-propanol | Cromoglicinsäure |
|---|---|---|
| Diplococcus pneumoniae | 50 | > 100 |
| Staphylococcus aureus 30–8 | 12,5 | > 100 |
| Staphylococcus aureus 30–3 | 12,5 | > 100 |
| Staphylococcus aureus 30–2 | 25 | > 100 |
| Staphylococcus aureus 30–64 | 50 | > 100 |
| Staphylococcus aureus 30–107 | 50 | > 100 |
| Staphylococcus aureus 30–109 | 25 | > 100 |
| Staphylococcus aureus 30–53 | 50 | > 100 |

Aufgrund ihrer pharmakologischen Eigenschaften eignen sich die Chinoloncarbonsäure-Derivate der allgemeinen Formel I gemäss Anspruch 1 in Kombination mit den in der galenischen Pharmazie üblichen Trägermitteln und Hilfsstoffen vorzugsweise zur Behandlung entzündlicher und allergischer Erkrankungen der Atemwege, wie zum Beispiel des Bronchialasthmas oder der Rhinitis.

Die Herstellung der Arzneimittelspezialitäten erfolgt in üblicher Weise, indem man die Wirkstoffe mit geeigneten Zusätzen, Trägersubstanzen und Geschmackskorrigentien in die gewünschten Applikationsformen wie Tabletten, Dragees, Kapseln, Lösungen oder vorzugsweise Inhalationsmitteln überführt.

Zur Herstellung von Inhalationsmitteln werden die Chinoloncarbonsäure-Derivate in an sich bekannter Weise pulverisiert oder in einem geeigneten Lösungsmittel gelöst oder suspendiert und gegebenenfalls mit geeigneten Zusätzen, wie Verdünnungsmittel, Verdickungsmittel, Suspensionshilfsmitteln, Treibgasen, Geschmackskorregentien, versetzt. Als Wirkstoffe verwendet man für die Inhalationsmittel üblicherweise ein Chinoloncarbonsäure-Derivat oder eine Mischung von zwei Chinoloncarbonsäure-Derivaten, es ist aber auch möglich, Inhalationsmittel zu formulieren, welche neben dem Chinoloncarbonsäure-Derivat zusätzlich noch andere Wirkstoffe, etwa Antibiotika, wie zum Beispiel Chloramphenicol, Tetracycline, Penicilline, Cephalosporine, Lincomycine, Erythromycine oder Rhifamycine, oder vorzugsweise auch Bronchodilatoren wie zum Beispiel Orciprenalin, Isoätharin oder insbesondere Isoprenalin (als Salz vorzugsweise als Sulfat) enthalten.

Ferner ist es auch möglich, die Wirkstoffe in einem physiologisch verträglichen Lösungsmittel, wie zum Beispiel Wasser oder Alkohol, zu lösen oder zu suspendieren und dann gegebenenfalls mit den üblichen Zusätzen zu versetzen.

Die so erhaltenen Lösungen oder Suspensionen, welche vorzugsweise 0,01 bis 10% Wirkstoff enthalten, können mit Hilfe der üblichen Inhalatoren appliziert werden.

Anderseits kann man die Wirkstoffe gegebenenfalls mit den üblichen Zusätzen in einem physiologisch verträglichen Treibgas, wie zum Beispiel Frigen® suspendieren oder lösen und die so erhaltenen Suspensionen oder Lösungen in Sprühdosen, die vorzugsweise mit einem Dosierventil versehen sind, abfüllen. Die so erhaltenen Inhalationsmittel, welche vorzugsweise 0,01 bis 10% Wirkstoff enthalten, werden in üblicher Weise appliziert.

Für die Trockenzerstäubung ist es besonders vorteilhaft, die Wirkstoffe durch Mikronisieren

oder Ausfällen auf eine mittlere Korngrösse von 0,01 bis 10 µ zu bringen.

Zur Erhöhung der Lagerfähigkeit und zur Erleichterung der Aerosolbildung setzt man diesen Kortikoid-Pulvern zweckmässigerweise noch zusätzlich einen festen, pharmakologisch unwirksamen wasserlöslichen pulverförmigen Träger mit einer mittleren Korngrösse von 20 bis 400 µ zu. Als Träger eignen sich beispielsweise Dextran, Mannit, Glucose oder Laktose. Diese pulverförmigen Inhalationsmittel, welchen zusätzlich noch weitere Zusätze, wie zum Beispiel Geschmackskorregentien (zum Beispiel Saccharin) oder Bronchodilatoren (zum Beispiel Isoprenalinsulfat) zugefügt werden können, enthalten üblicherweise 0,1 bis 30% Wirkstoff. Die Herstellung von pulverförmigen Inhalationsmitteln und ihre Anwendung ist beispielsweise in der britischen Patentschrift 1 144 906 beschrieben.

Die erfindungsgemässen Inhalationsmittel werden, wie bereits erwähnt, vorzugsweise zur Behandlung von allergischen Erkrankungen der Atemwege, wie zum Beispiel rhinitische Erkrankungen, Heuschnupfen oder Bronchialasthma, verwendet.

Die Menge des Inhalationsmittels, welches pro Inhalation zu applizieren ist, variiert nach der Schwere der Erkrankung und der Konstitution des zu Behandelnden. Üblicherweise wird man pro Inhalation etwa 0,1 bis 100 mg und vorzugsweise 1 bis 50 mg Chinoloncarbonsäure-Derivat gegebenenfalls in Kombination mit 0,01 bis 10 mg Bronchodilator appliziert.

Die Chinoloncarbonsäure-Derivate der allgemeinen Formel I gemäss Anspruch 1 können nach dem in Anspruch 16 gekennzeichneten Verfahren hergestellt werden.

Das Verfahren gemäss Verfahrensvariante a wird in an sich bekannter Weise durchgeführt (Jucker (Ed), Fortschritte Arzneimittelforschung Bd 21, 1977, Seite 1f). So kann man beispielsweise die Diamine der allgemeinen Formel II mit den Malonsäure-Derivaten der Formel III umsetzen und erhält die Verbindungen der allgemeinen Formel IV. Für diese Reaktion ist die Anwesenheit von Katalysatoren oder Lösungsmitteln nicht erforderlich, es ist aber selbstverständlich auch möglich, diese Reaktion in Gegenwart zusätzlicher Lösungsmittel wie zum Beispiel Chlorkohlenwasserstoffe (Methylenchlorid, Chloroform, Tetrachloräthan usw.) Äther (Diisopropyläther, Dibutyläther, Dioxan, Tetrahydrofuran usw.) oder dipolaren aprotischen Lösungsmitteln (Dimethylformamid, Hexamethylphosphorsäuretriamid usw.) durchzuführen. Dieser Reaktionsschrittt wird vorzugsweise bei einer Reaktionstemperatur von 20 bis 150 °C durchgeführt.

Die sich an diesen Verfahrensschritt anschliessende Cyclisierung der Verbindungen der allgemeinen Formel IV erfolgt thermisch, vorzugsweise in Gegenwart hochsiedender Lösungsmittel (wie zum Beispiel Mineralöl, Diphenyl, Diphenyläther oder Dawtherm®A) bei 200 bis 350 °C. Bei dieser Reaktion kann man die Reaktionstemperatur auf ca. 50 bis 200 °C senken, wenn man dem Reaktionsgemisch als Katalysator zusätzlich eine Lewis-Säure (Essigsäure, Schwefelsäure, Polyphosphorsäure, Bortrifluorid, Phosphoroxychlorid, Phosphortrichlorid, Phosphorpentachlorid, Aluminiumchlorid, Zinkchlorid usw.) zusetzt.

Bei dem erfindungsgemässen Verfahren gemäss Verfahrensvariante b werden die Hydroxychinoloncarbonsäure-Derivate der allgemeinen Formel V in inerten Lösungsmitteln und in Gegenwart basischer Katalysatoren mit den Verbindungen der allgemeinen Formel VI oder VII kondensiert. Diese Reaktion wird vorzugsweise mit solchen Hydroxychinoloncarbonsäure-Derivaten der allgemeinen Formel V durchgeführt, in denen der Substituent $R_1$ einen Alkylrest darstellt. Für diese Reaktion kann man als basische Katalysatoren beispielsweise Alkalimetallhydrogenkarbonate (Natriumhydrogenkarbonat, Kaliumhydrogenkarbonat), Alkalikarbonate (Natriumkarbonat, Kaliumkarbonat), Alkalimetallhydroxide (Natriumhydroxid, Kaliumhydroxid) oder Alkalimetallalkoholate (Natriummethylat, Natriumäthylat, Kaliummethylat, Kaliumtertiärbutylat usw.) anwenden. Geeignete Lösungsmittel sind beispielsweise niedere Alkohole (insbesondere solche mit 1 bis 4 Kohlenstoffatomen wie Methanol, Äthanol, Propanol, Isopropanol oder tert.-Butanol), polare Äther (Dioxan, Tetrahydrofuran, Glykoldimethyläther, Glykolmonomethyläther usw.) oder dipolare aprotische Lösungsmittel (wie Dimethylformamid, N-Methylacetamid, Dimethylsulfoxid oder Hexamethylphosphorsäuretriamid).

Die gewünschtenfalls nachfolgende Verseifung der Ester erfolgt nach an sich bekannten Arbeitsmethoden. Beispielsweise genannt sei die Verseifung der Ester in Wasser oder wässrigen Alkoholen in Gegenwart von sauren Katalysatoren, wie Salzsäure, Schwefelsäure, p-Toluolsulfonsäure oder von basischen Katalysatoren wie Kaliumhydrogencarbonat, Kaliumcarbonat, Natriumhydroxid oder Kaliumhydroxid.

Die sich gewünschtenfalls anschliessende Alkylierung der eventuell vorhandenen sekundären Aminogruppen erfolgt ebenfalls nach den bekannten Methoden, die man üblicherweise zur N-Alkylierung von Chinolin-Derivaten verwendet.

So kann man beispielsweise die Stickstoffatome der Verbindungen durch Umsetzung mit Metallhydriden oder Metallamiden – wie Natriumhydrid oder Natriumamid – metallisieren und auf die so erhaltenen reaktiven Verbindungen die Sulfate oder die Halogenide (Chloride, Bromide oder Jodide) des letztlich gewünschten Kohlenwasserstoffrestes einwirken lassen. Für diese Reaktion, die bei einer Reaktionstemperatur von etwa 0 bis 120 °C durchgeführt wird, werden vorzugsweise polare aprotische Lösungsmittel wie Dimethylformamid, N-Methylpyrrolidon oder Hexymethylphosphorsäuretriamid verwendet.

Die sich als Gewünschtenfallsmassnahme anschliessende Veresterung der freien Hydroxymethylgruppe erfolgt ebenfalls nach den dafür bekannten Arbeitsmethoden. Als mögliche Veresterungsmethode sei beispielsweise die Veresterung der Hydroxyverbindungen mit Säureanhydriden oder Säurechloriden in Gegenwart aromatischer N-Heterocyclen wie Pyridin, Collidin oder Lutidin oder in Gegenwart wässriger Lösungen von basischen Alkalimetallbonat, Natriumbicarbonat, Kaliumbicarbonat, Natriumcarbonat, Natriumhydroxyd oder Kaliumhydroxyd genannt.

Die sich als Gewünschtenfallsmassnahme anschliessende Überführung der Chinoloncarbonsäure-Derivate in ihre Salze erfolgt ebenfalls unter den dem Fachmann wohlbekannten Bedingungen, indem man diese Verbindungen mit einer physiologisch unbedenklichen Base neutralisiert.

Die Ausgangsverbindungen für das erfindungsgemässe Verfahren sind bekannt oder können in an sich bekannter Weise hergestellt werden. Nachfolgend ist das Herstellungsverfahren für diese Ausgangsverbindungen an Hand einiger typischer Verbindungen beschrieben:

A) 1-Butyl-1,4-dihydro-7-methoxy-4-chinolon-3-carbonsäure

11,0 g (50 mMol) 4-Hydroxy-7-methoxy-chinolin-3-carbonsäure werden in eine Suspension aus 14,0 g (351 mMol) pulverisierten Natriumhydroxids in 200 ml Dimethylsulfoxid gegeben. Es wird 10 Minuten bei Raumtemperatur gerührt, dann werden 16,1 ml (150 mMol) Butylbromid zugetropft, so dass die Temperatur 25 °C nicht überschreitet. Anschliessend wird 2 Stunden bei Raumtemperatur gerührt, in Wasser gegossen und mit Salzsäure angesäuert. Das ausgefallene Produkt wird abfiltriert, mit Wasser gewaschen, getrocknet und aus Dimethylformamid umkristallisiert.
Schmelzpunkt: 160–162 °C. Ausbeute: 9,18 g.

B) 1-Butyl-1,4-dihydro-7-hydroxy-4-chinolon-3-carbonsäure

5,0 g (18 mMol) 1-Butyl-1,4-dihydro-7-methoxy-4-chinolon-3-carbonsäure werden in 65 ml Bromwasserstoffsäure (48%) 4 Stunden unter Rückfluss gekocht. Anschliessend wird in Wasser gegossen, das Festprodukt abfiltriert und aus Dimethylformamid umkristallisiert.
Schmelzpunkt: 228–240 °C. Ausbeute: 3,5 g.

C) 1,4-Dihydro-7-hydroxy-1-methyl-4-chinolon-3-α-carbonsäure

5,83 g (25 mMol) 1,4-Dihydro-7-methoxy-1-methyl-4-chinolon-3-carbonsäure werden mit 65 ml Bromwasserstoffsäure 4 Stunden unter Rückfluss gekocht. Es wird in Wasser gegossen, das Festprodukt abfiltriert und aus Essigsäure umkristallisiert.
Schmelzpunkt: 285–294 °C. Ausbeute: 5,6 g.

D) 1,3-Bis-(3-Nitrophenyloxy)-2-propanol

44,3 g (0,32 mMol) 3-Nitrophenol werden in 1,2 l Isopropanol gelöst, 13,7 ml Epichlorhydrin zugegeben und anschliessend eine Lösung von 17,5 g Kaliumhydroxid in 250 ml Isopropanol sowie 10 ml Wasser zugesetzt. Das Gemisch wird 48 Stunden unter Rückfluss gekocht, auf das halbe Volumen eingeengt und mit 250 ml Wasser versetzt. Das ausgefallene Produkt wird abfiltriert, mit Wasser gewaschen und getrocknet.
Schmelzpunkt: 110 °C. Ausbeute: 24 g.

E) 1,3-Bis-(4-Nitrophenyloxy)-2-propanol

8,86 g (63,7 mMol) 4-Nitrophenol werden in 250 ml Isopropanol gelöst, 2,75 ml (35,1 mMol) Epichlorhydrin zugegeben und anschliessend eine Lösung von 1,98 g (35,3 mMol) Kaliumhydroxid in 25 ml Isopropanol sowie 1 ml Wasser zugesetzt. Das Gemisch wird 48 Stunden unter Rückfluss gekocht, auf das halbe Volumen eingeengt und mit 50 ml Wasser versetzt. Das ausgefallene Produkt wird abfiltriert und aus Isopropanol/Äthanol 1 : 1 umkristallisiert.
Schmelzpunkt: 144–145 °C. Ausbeute: 6,4 g.

Die nachfolgenden Ausführungsbeispiele dienen zur näheren Erläuterung der Erfindung.

Beispiel 1

1,3-Bis-(1-äthyl-3-carboxy-1,4-dihydro-4-oxo-7-chinolyloxy)-2-propanol

a) 0,46 g (20 mMol) Natrium werden in 20 ml Äthanol gegeben. Nach Beendigung der Umsetzung werden in der erhaltenen Natriumäthylat-Lösung 2,33 g (10 mMol) 1-Äthyl-1,4-dihydro-7-hydroxy-4-chinolon-3-carbonsäure gelöst, anschliessend 0,91 ml (11,7 mMol) Epichlorhydrin und eine Spur Kaliumjodid zugegeben. Das Gemisch wird 8 Stunden unter Rückfluss gekocht, eingedampft und der Rückstand in Wasser gelöst. Die Lösung wird mit Chloroform und Essigester ausgeschüttelt und mit Salzsäure bis auf pH 5 angesäuert. Das ausgefallene Produkt wird abfiltriert, mit Wasser gewaschen, getrocknet und in heissem Dimethylformamid gelöst. Zur Kristallisation wird mit Isopropyläther versetzt. Das erhaltene Material wird bei 100 °C im Vakuum getrocknet.
Schmelzpunkt: 305–310 °C. Ausbeute: 1,26 g.

b) 0,17 g (3,0 mMol) pulverisiertes Kaliumhydroxid werden in 5 ml Dimethylsulfoxid suspendiert und 0,10 g (0,22 mMol) 1,3-Bis-(3-Carboxy-1,4-dihydro-4-oxo-7-chinolyloxy)-2-propanol eingetragen. Es wird 10 Minuten bei Raumtemperatur gerührt, dann werden 0,17 ml Diäthylsulfat zugetropft. Es wird 2 Stunden bei Raumtemperatur gerührt, dann in Wasser gegeben und angesäuert. Das erhaltene Festprodukt wird abfiltriert, mit Wasser gewaschen, mit Essigsäure ausgekocht und Dimethylformamid/Isopropyläther umkristallisiert.
Schmelzpunkt: 305–310 °C. Ausbeute: 0,05 g.

Beispiel 2

1,3-Bis-(1-butyl-3-carboxy-1,4-dihydro-4-oxo-7-chinolyl-oxy)-2-propanol

0,096 g (4,2 mMol) Natrium werden in 5 ml Äthanol gegeben, anschliessend 0,56 g (2,1 mMol) 1-Butyl-1,4-dihydro-7-hydroxy-4-chinolon-3-carbonsäure, 0,19 ml Epichlorhydrin und eine Spur Kaliumjodid. Es wird 4 Stunden unter Rückfluss gekocht, anschliessend das Reaktionsgemisch mit 2n NaOH vollständig in Lösung gebracht und

dann mit Salzsäure angesäuert. Das ausgefällte Produkt wird abfiltriert, mit Wasser gewaschen und getrocknet.
Schmelzpunkt: 255–260 °C. Ausbeute: 0,45 g.

Beispiel 3
1,3-Bis-(3-carboxy-1,4-dihydro-1-methyl-4-oxo-7-chinolyl-oxy)-2-propanol

0,46 g (20 mMol) Natrium werden in 20 ml Methanol gegeben, anschliessend 2,00 g (9,1 mMol) 1,4-Dihydro-7-hydroxy-1-methyl-4-chinolon-3-carbonsäure, 1,21 ml (15,5 mMol) Epichlorhydrin und eine Spur Kaliumjodid. Es wird 8 Stunden unter Rückfluss gekocht, eingedampft und der Rückstand in Wasser gelöst. Die Lösung wird mit Chloroform ausgeschüttelt und mit Salzsäure angesäuert. Das ausgefällte Produkt wird abfiltriert, mit Wasser gewaschen und getrocknet.
Schmelzpunkt: 310 °C (Zersetzung). Ausbeute: 0,82 g.

Beispiel 4
1,3-Bis-(3-carboxy-1,4-dihydro-4-oxo-7-chinolyloxy)-2-propanol

10 g (30 mMol) 1,3-Bis-(3-nitrophenyloxy)-2-propanol werden in 250 ml Methanol gelöst und in Gegenwart von 1 g Pd-Kohle (10%) hydriert. Der Katalysator wird abfiltriert und die Lösung eingedampft, es hinterbleiben 8,2 g rohes 1,3-Bis-(3-aminophenyloxy)-2-propanol. Dieses wird mit 100 ml Äthoxymethylenmalonsäurediäthylester 3 Stunden auf 100 °C erhitzt, anschliessend 1 Stunde bei 100 °C im Vakuum einer Wasserstrahlpumpe. Der überschüssige Äthoxymethylenmalonsäureäthylester wird im Vakuum abdestilliert, der Rückstand in 100 ml Dowtherm A® 30 Minuten unter Rückfluss gekocht. Nach dem Abkühlen wird mit Isopropyläther versetzt. Das abgeschiedene rohe 1,3-Bis-(3-äthoxycarbonyl-1,4-dihydro-4-oxo-chinolyloxy)-2-propanol wird abgetrennt, in 150 ml 2n NaOH suspendiert und 2 Stunden unter Rückfluss gekocht. Nach Abkühlen wird die Lösung mit Essigester ausgeschüttelt, mit Salzsäure angesäuert und mit etwas Methanol versetzt. Das erhaltene kristalline Material wird abfiltriert, mit Wasser gewaschen und getrocknet.
Schmelzpunkt: > 300 °C. Ausbeute: 3,2 g.

Beispiel 5
1,3-Bis-(3-carboxy-1,4-dihydro-4-oxo-6-chinolyloxy)-2-propanol

10 g (30 mMol) 1,3-Bis-(4-nitrophenyloxy)-2-propanol werden in Methanol in Gegenwart von 1,6 g Pd/Kohle (10%) hydriert. Der Katalysator wird abfiltriert und das Filtrat eingedampft. Es hinterbleiben 13 g rohes, öliges 1,3-Bis-(4-aminophenyloxy)-2-propanol, dieses wird mit 100 ml Äthoxymethylenmalonsäurediäthylester 3 Stunden auf 100 °C erhitzt, anschliessend 1 Stunde auf 100 °C im Wasserstrahlpumpenvakuum. Der Überschuss des Äthoxymethylenmalonsäurediäthylesters wird im Vakuum abdestilliert, der Rückstand in 150 ml Dowtherm A® 30 Minuten unter Rückfluss gekocht. Nach Abkühlen wird die Lösung mit Isopropyläther versetzt und das abgeschiedene Festprodukt abfiltriert. Es werden 4 g rohes 1,3-Bis-(3-carbäthoxy-1,4-dihydro-4-oxo-6-chinolyloxy)-2-propanol erhalten, welches in 100 ml 2n NaOH 1 Stunde unter Rückfluss gekocht wird. Nach Abkühlen wird mit Essigester ausgeschüttelt und die wässrige Phase mit Salzsäure angesäuert. Das ausgeschiedene ölige Produkt wird abgetrennt und mit Äther verrührt, bis es fest geworden ist. Das Festprodukt wird abfiltriert, aus Dimethylformamid umkristallisiert und bei 100 °C im Vakuum getrocknet.
Schmelzpunkt: > 300 °C. Ausbeute: 1,4 g.

Beispiel 6
1,3-Bis-(1-äthyl-3-carboxy-1,4-dihydro-4-oxo-6-chinolyl-oxy)-2-propanol

1,5 g (6,4 mMol) 1-Äthyl-1,4-dihydro-6-hydroxy-4-chinolon-3-carbonsäure werden in eine Lösung aus 0,29 g (12,6 mMol) Natrium in 15 ml Äthanol gegeben, anschliessend werden 0,58 ml (7,5 mMol) Epichlorhydrin zugesetzt und 20 mg Kaliumjodid. Das Gemisch wird 4 Stunden unter Rückfluss gekocht, mit 2n NaOH bis zur klaren Lösung versetzt, dann mit 2n HC1 angesäuert. Das ausgefällte Produkt wird abfiltriert, aus Essigsäure umkristallisiert und bei 120 °C im Vakuum getrocknet.
Schmelzpunkt: > 300 °C. Ausbeute: 1,3 g.

Beispiel 7
1,3-Bis-(1-äthyl-3-carboxy-1,4-dihydro-4-oxo-8-chinolyl-oxy)-2-propanol

0,466 g (2 mMol) 1-Äthyl-1,4-dihydro-8-hydroxy-4-chinolon-3-carbonsäure werden in 10 ml Isopropanol suspendiert, 0,086 ml (1,1 mMol) Epichlorhydrin zugegeben, anschliessend eine Lösung aus 0,224 g (4,0 mMol) Kaliumhydroxid in 5 ml Isopropanol und 2 Tropfen Wasser. Das Gemisch wird 48 Stunden unter Rückfluss gekocht, mit 2n NaOH versetzt bis zur klaren Lösung, dann wird mit 2n HC1 neutralisiert. Das ausgefällte Material wird abfiltriert, mit Wasser gewaschen und im Vakuum getrocknet.
Schmelzpunkt: 250 °C (Zersetzung). Ausbeute: 0,2 g.

Beispiel 8
2-Acetoxy-1,3-bis-(1-äthyl-3-carboxy-1,4-dihydro-4-oxo-7-chinolyloxy)-propan

1,0 g (4,3 mMol) 1,3-Bis-(1-äthyl-3-carboxy-1,4-dihydro-4-oxo-7-chinolyloxy)-2-propanol werden in einem Gemisch aus 7,5 ml Acetanhydrid und 7,5 ml Essigsäure 1 Stunde auf 100 °C erhitzt. Anschliessend wird in Wasser gegossen, das ausgefallene Festprodukt abfiltriert, mit Wasser gewaschen und bei 100 °C im Vakuum getrocknet.
Schmelzpunkt: 144–147 °C. Ausbeute: 1,0 g.

Beispiel 9
1,3-Bis-(1-äthyl-3-carboxy-1,4-dihydro-4-oxo-7-chinolyloxy)-2-propanol, Dinatriumsalz

0,52 g (1,0 mMol) 1,3-Bis-(1-äthyl-3-carboxy-1,4-dihydro-4-oxo-7-chinolyloxy)-2-propanol werden in einer Mischung aus 2 ml 1n NaOH-Lösung und 20 ml Methanol bei Raumtemperatur aufgelöst.

Anschliessend wird im Vakuum eingedampft und der Rückstand unter Entfärben mit Aktivkohle aus Wasser umkristallisiert.
Schmelzpunkt: 315 °C (Zersetzung). Ausbeute: 0,39 g.

Beispiel 10
Zusammensetzung eines Aerosalz

1,3-Bis-(1-äthyl-3-carboxy-1,4-dihydro-4-oxo-7-chinolyloxy)-2-propanol,

| | | |
|---|---|---|
| Dinatriumsalz | 2,00 | % |
| Isoprenalinsulfat | 0,10 | % |
| Natrium-dicetylsulfosuccinat | 0,004 | % |
| Mischung (60 : 40) von Propellant 12 und Propellant 14 | ad 100,00 | % |

Beispiel 11
Zusammensetzung einer Pulverformulierung

1,3-Bis-(1-äthyl-3-carboxy-1,4-dihydro-4-oxo-7-chinolyloxy)-2-propanol,

| | | |
|---|---|---|
| Dinatriumsalz, mikronisiert 2–8 µ | 20 | mg |
| Isoprenalinsulfat, mikronisiert 2–8 µ | 0,1 | mg |
| Lactose, gepulvert 80–120 µ | 15 | mg |

Angaben gelten für eine Dosiseinheit.

**Patentansprüche für die Vertragsstaaten: BE, CH, DE, FR, GB, IT, LU, NL, SE**

1. Chinoloncarbonsäure-Derivate der allgemeinen Formel I

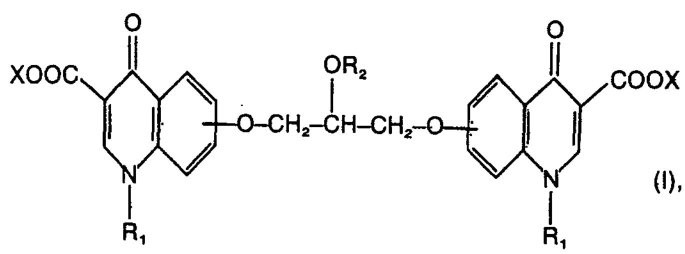

(I),

worin die Gruppierung

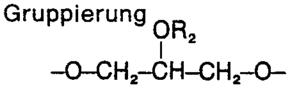

sich in der 6-, 7-, oder 8-Position der beiden Chinolonreste befindet und worin

$R_1$ ein Wasserstoffatom oder eine 1 bis 6 Kohlenstoffatome enthaltende Alkylgruppe,

$R_2$ ein Wasserstoffatom oder eine 1 bis 8 Kohlenstoffatome enthaltende Acylgruppe und

X ein Wasserstoffatom, das Kation einer physiologisch unbedenklichen Base oder eine 1 bis 6 Kohlenstoffatome enthaltende Alkylgruppe bedeuten.

2. Chinoloncarbonsäure-Derivate der allgemeinen Formel I gemäss Anspruch 1, dadurch gekennzeichnet, dass die Gruppierung

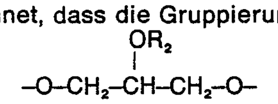

sich jeweils in der 6-Position, oder jeweils in der 7-Position, oder jeweils in der 8-Position der beiden Chinolonreste befindet.

3. Chinoloncarbonsäure-Derivate der allgemeinen Formel I gemäss Ansprüchen 1 und 2, dadurch gekennzeichnet, dass $R_2$ ein Wasserstoffatom bedeutet.

4. Chinoloncarbonsäure-Derivate der allgemeinen Formel I gemäss Ansprüchen 1 bis 3, dadurch gekennzeichnet, dass X ein Wasserstoffatom, ein Alkalimetallkation, ein Erdalkalimetallkation, ein Ammoniumkation, ein Piparazinylkation oder ein N-Methylglucaminkation bedeutet.

5. 1,3-Bis-(1-äthyl-3-carboxy-1,4-dihydro-4-oxo-7-chinolyloxy)-2-propanol.

6. 1,3-Bis-(1-butyl-3-carboxy-1,4-dihydro-4-oxo-7-chinolyloxy)-2-propanol.

7. 1,3-Bis-(3-carboxy-1,4-dihydro-1-methyl-4-oxo-7-chinolyloxy)-2-propanol.

8. 1,3-Bis-(3-carboxy-1,4-dihydro-4-oxo-7-chinolyloxy)-2-propanol.

9. 1,3-Bis-(3-carboxy-1,4-dihydro-4-oxo-6-chinolyloxy)-2-propanol.

10. 1,3-Bis-(1-äthyl-3-carboxy-1,4-dihydro-4-oxo-6-chinolyloxy)-2-propanol.

11. 1,3-Bis-(1-äthyl-3-carboxy-1,4-dihydro-4-oxo-8-chinolyloxy)-2-propanol.

12. 2-Acetoxy-1,3-bis-(1-äthyl-3-carboxy-1,4-dihydro-4-oxo-7-chinolyloxy)-propan.

13. Dinatriumsalz des 1,3-Bis-(1-äthyl-3-carboxy-1,4-dihydro-4-oxo-7-chinolyloxy)-2-propanol.

14. Pharmazeutische Präparate gekennzeichnet durch den Gehalt eines Chinoloncarbonsäure-Derivates gemäss Ansprüchen 1 bis 13.

15. Verfahren zur Herstellung von Chinoloncarbonsäure-Derivaten gemäss Anspruch 1, dadurch gekennzeichnet, dass man

a) ein Diamin der allgemeinen Formel II

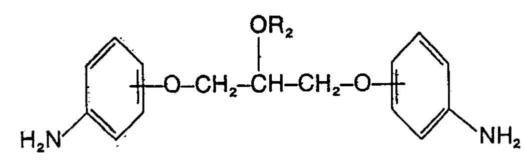

(II),

worin

$R_2$ die im Anspruch 1 genannte Bedeutung besitzt, mit einem Malonsäure-Derivat der allgemeinen Formel III

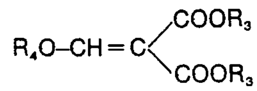

(III),

worin

$R_3$ und $R_4$ jeweils 1 bis 6 Kohlenstoffatome enthaltende Alkylgruppen darstellen, kondensiert, die erhaltene Verbindung der allgemeinen Formel IV

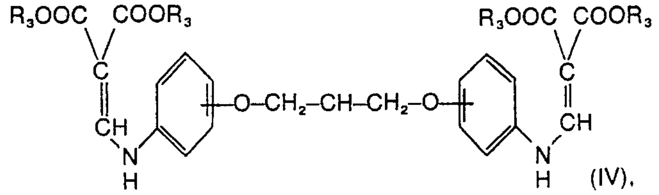

(IV),

worin

$R_2$ und $R_3$ die obengenannte Bedeutung besitzen, cyclisiert, oder

b) ein Hydroxychinoloncarbonsäure-Derivat der allgemeinen Formel V

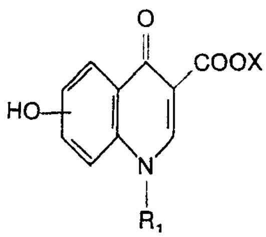

(V),

worin

X und $R_1$ die im Anspruch 1 genannte Bedeutung besitzen und die HO-Gruppe in der 6-, 7- oder 8-Position des Chinolonkernes ständig ist, mit einer Verbindung der allgemeinen Formel VI oder VII

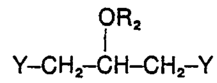

(VI),

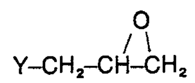

(VII),

worin

$R_2$ die in Anspruch 1 genannte Bedeutung besitzt und Y ein Chloratom, ein Bromatom, ein Jodatom, ein Mesylatrest oder Tosylatrest darstellt, kondensiert, und gewünschtenfalls die gemäss Verfahrensvariante a oder b erhaltenen Chinoloncarbonsäure-Derivate verseift, verestert, N-alkyliert oder mit physiologisch unbedenklichen Basen in ihre Salze überführt.

**Patentanspruch für den Vertragsstaat: AT**

Verfahren zur Herstellung von Chinoloncarbonsäure-Derivaten der allgemeinen Formel I

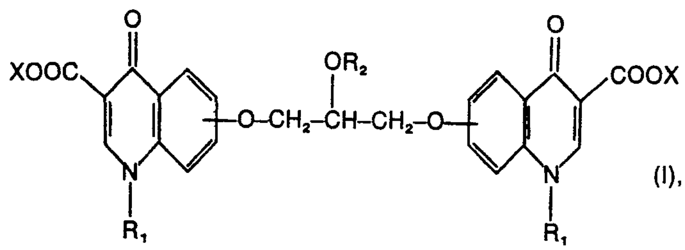

(I),

worin die Gruppierung

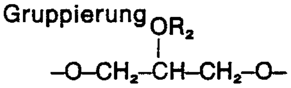

sich in der 6-, 7- oder 8-Position der beiden Chinolonreste befindet und worin

$R_1$ ein Wasserstoffatom oder eine 1 bis 6 Kohlenstoffatome enthaltende Alkylgruppe,

$R_2$ ein Wasserstoffatom oder eine 1 bis 8 Kohlenstoffatome enthaltende Acylgruppe und

X ein Wasserstoffatom, das Kation einer physiologisch unbedenklichen Base oder eine 1 bis 6 Kohlenstoffatome enthaltende Alkylgruppe bedeuten, dadurch gekennzeichnet, dass man

a) ein Diamin der allgemeinen Formel II

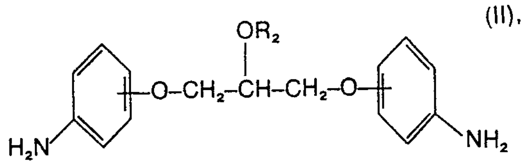

(II),

worin

$R_2$ die im Anspruch 1 genannte Bedeutung besitzt, mit einem Malonsäure-Derivat der allgemeinen Formel III

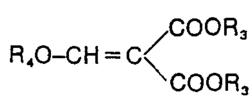

(III),

worin

$R_3$ und $R_4$ jeweils 1 bis 6 Kohlenstoffatome enthaltende Alkylgruppen darstellen, kondensiert, die erhaltene Verbindung der allgemeinen Formel IV

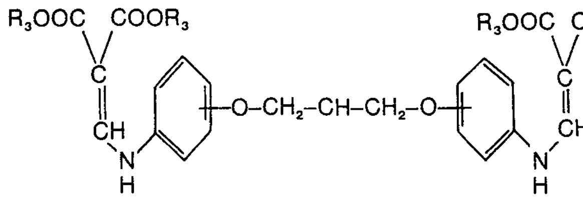
(IV),

worin

$R_2$ und $R_3$ die obengenannte Bedeutung besitzen, cyclisiert, oder

b) ein Hydroxychinoloncarbonsäure-Derivat der allgemeinen Formel V

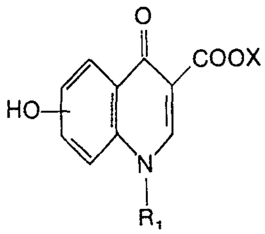
(V),

worin

X und $R_1$ die im Anspruch 1 genannte Bedeutung besitzen und die HO-Gruppe in der 6-, 7- oder 8-Position des Chinolonkernes ständig ist, mit einer Verbindung der allgemeinen Formel VI oder VII

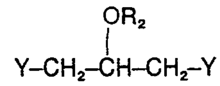
(VI),

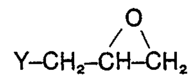
(VII),

worin

$R_2$ die in Anspruch 1 genannte Bedeutung besitzt und

Y ein Chloratom, ein Bromatom, ein Jodatom, ein Mesylatrest oder Tosylatrest darstellt, kondensiert, und gewünschtenfalls die gemäss Verfahrensvariante a oder b erhaltenen Chinoloncarbonsäure-Derivate verseift, verestert, N-alkyliert oder mit physiologisch unbedenklichen Basen in ihre Salze überführt.

**Claims for the contracting states: BE, CH, DE, FR, GB, IT, LU, NL, SE**

1. Quinolonecarboxylic acid derivatives of the general formula I

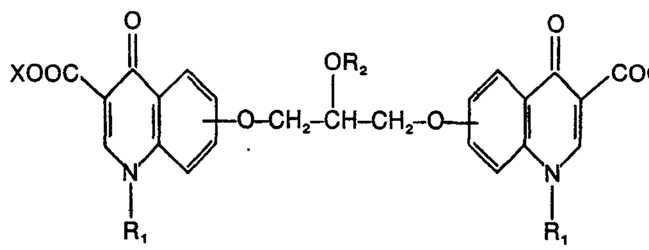
(I),

in which the

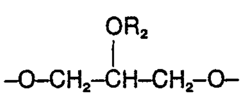

grouping is in the 6-, 7- or 8-position of the two quinolone radicals, and in which

$R_1$ represents a hydrogen atom or an alkyl group containing 1 to 6 carbon atoms,

$R_2$ represents a hydrogen atom or an acyl group containing 1 to 8 carbon atoms, and

X represents a hydrogen atom, the cation of a physiologically tolerable base or an alkyl group containing 1 to 6 carbon atoms.

2. Quinolonecarboxylic acid derivatives of the general formula I according to claim 1, characterised in that the

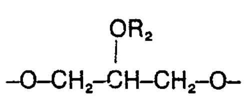

grouping is in each case in the 6-position, in each case in the 7-position, or in each case in the 8-position of the two quinolone radicals.

3. Quinolonecarboxylic acid derivatives of the general formula I according to claims 1 and 2, characterised in that $R_2$ represents a hydrogen atom.

4. Quinolonecarboxylic acid derivatives of the general formula I according to claims 1 to 3, characterised in that X represents a hydrogen atom, an alkali metal cation, an alkaline earth metal cation, an ammonium cation, a piperazinyl cation or an N-methylglucamine cation.

5. 1,3-bis-(1-ethyl-3-carboxy-1,4-dihydro-4-oxo-7-quinolyloxy)-propan-2-ol.

6. 1,3-bis-(1-butyl-3-carboxy-1,4-dihydro-4-oxo-7-quinolyloxy)-propan-2-ol.

7. 1,3-bis-(3-carboxy-1,4-dihydro-1-methyl-4-oxo-7-quinolyloxy)-propan-2-ol.

8. 1,3-bis-(3-carboxy-1,4-dihydro-4-oxo-7-quinolyloxy)-propan-2-ol.

9. 1,3-bis-(3-carboxy-1,4-dihydro-4-oxo-6-quinolyloxy)-propan-2-ol.

10. 1,3-bis-(1-ethyl-3-carboxy-1,4-dihydro-4-oxo-6-quinolyloxy)-propan-2-ol.

11. 1,3-bis-(1-ethyl-3-carboxy-1,4-dihydro-4-oxo-8-quinolyloxy)-propan-2-ol.

12. 2-acetoxy-1,3-bis-(1-ethyl-3-carboxy-1,4-dihydro-4-oxo-7-quinolyloxy)-propane.

13. Disodium salt of 1,3-bis-(1-ethyl-3-carboxy-1,4-dihydro-4-oxo-7-quinolyloxy)-propan-2-ol.

14. Pharmaceutical preparations characterised by the content of a quinolonecarboxylic acid derivative according to claims 1 to 13.

15. Process for the manufacture of quinolonecarboxylic acid derivatives according to claim 1, characterised in that

a) a diamine of the general formula II

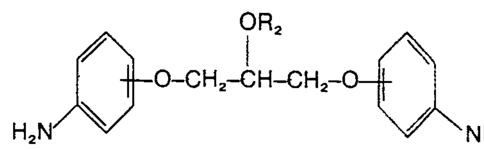
(II),

in which $R_2$ has the meaning given in claim 1, is condensed with a malonic acid derivative of the general formula III

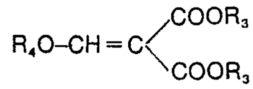
(III),

in which each of $R_3$ and $R_4$ represents an alkyl group containing 1 to 6 carbon atoms, and the resulting compound of the general formula IV

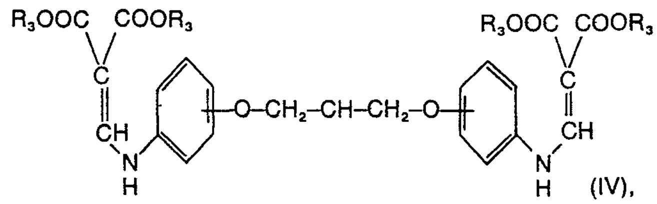
(IV),

in which $R_2$ and $R_3$ have the meanings given above, is cyclised, or

b) a hydroxyquinolonecarboxylic acid derivative of the general formula V

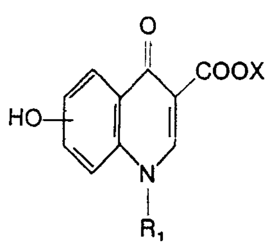
(V),

in which X and $R_1$ have the meanings given in claim 1 and the HO-group is in the 6-, 7- or 8-position of the quinolone nucleus, is condensed with a compound of the general formula VI or VII

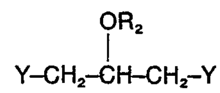
(VI),

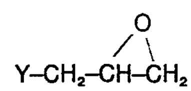
(VII),

in which $R_2$ has the meaning given in claim 1 and Y represents a chlorine atom, a bromine atom, an iodine atom, a mesylate radical or a tosylate radical, and if desired, the quinolonecarboxylic acid derivatives obtained according to process variant a or b are hydrolysed, esterified, N-alkylated or converted into their salts with physiologically tolerable bases.

**Claim for the contracting state AT**

Process for the manufacture of quinolonecarboxylic acid derivatives of the general formula I

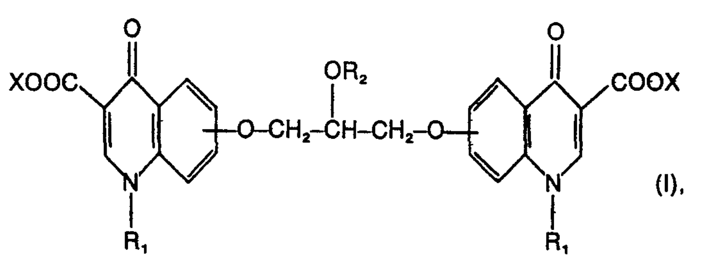
(I),

in which the

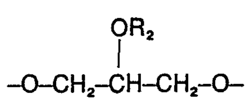

grouping is in the 6-, 7- or 8-position of the two quinolone radicals, and in which

$R_1$ represents a hydrogen atom or an alkyl group containing from 1 to 6 carbon atoms,

$R_2$ represents a hydrogen atom or an acyl group containing from 1 to 8 carbon atoms, and

X represents a hydrogen atom, the cation of a physiologically tolerable base or an alkyl group containing from 1 to 6 carbon atoms, characterised in that

a) a diamine of the general formula II

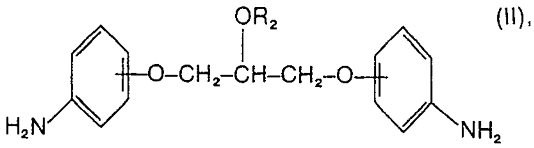

(II),

in which $R_2$ has the meaning given in claim 1, is condensed with a malonic acid derivative of the general formula III

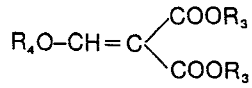

(III),

in which each of $R_3$ and $R_4$ represents an alkyl group containing 1 to 6 carbon atoms, and the resulting compound of the general formula IV

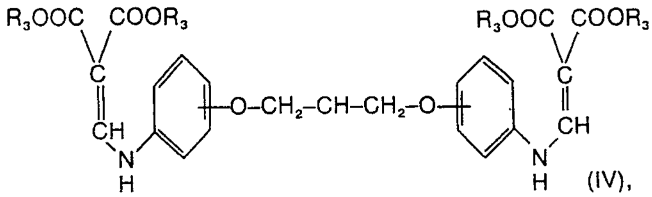

(IV),

in which $R_2$ and $R_3$ have the meanings given above, is cyclised, or

b) a hydroxyquinolonecarboxylic acid derivative of the general formula V

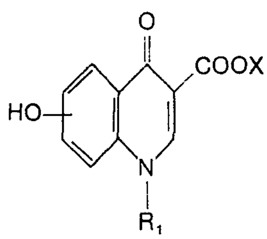

(V),

in which X and $R_1$ have the meanings given in claim 1 and the HO-group is in the 6-, 7- or 8-position of the quinolone nucleus, is condensed with a compound of the general formula VI or VII

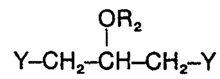

(VI),

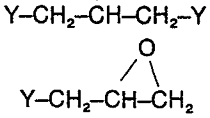

(VII),

in which $R_2$ has the meaning given in claim 1 and Y represents a chlorine atom, a bromine atom, an iodine atom, a mesylate radical or a tosylate radical, and if desired, the quinolonecarboxylic acid derivatives obtained according to process variant a or b are hydrolysed, esterified, N-alkylated or converted into their salts with physiologically tolerable bases.

**Revendications pour les Etats contractants: BE, CH, DE, FR, GB, IT, LU, NL, SE**

1) Dérivés d'acide quinolone-carboxylique de formule générale I

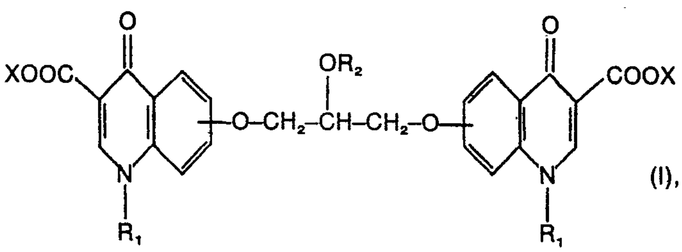

(I),

dans laquelle le groupement

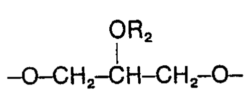

est à la position 6, 7 ou 8 de chacun des deux radicaux de quinolone et dans laquelle

$R_1$ représente un atome d'hydrogène ou un alkyle en $C_1$ à $C_6$,

$R_2$ un atome d'hydrogène ou un acyle en $C_1$ à $C_8$ et

X un atome d'hydrogène, le cation d'une base physiologiquement acceptable ou un alkyle en $C_1$ à $C_6$.

2) Dérivés selon la revendication 1, caractérisés en ce que le groupement

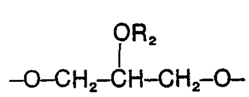

est situé à la position 6 de chacun des deux radicaux de quinolone, ou à la position 7 de chacun

des deux, ou à la position 8 de chacun des deux.

3) Dérivés selon la revendication 1 ou 2 dans lesquels $R_2$ est un atome d'hydrogène.

4) Dérivés selon l'une quelconque des revendications 1 à 3, caractérisés en ce que X est un atome d'hydrogène, un cation de métal alcalin, de métal alcalino-terreux ou d'ammonium, un cation pipérazinyle ou un cation de N-méthylglucamine.

5) 1,3-Bis-(1-éthyl-3-carboxy-1,4-dihydro-4-oxo-7-quinolyloxy)-2-propanol.

6) 1,3-Bis-(1-butyl-3-carboxy-1,4-dihydro-4-oxo-7-quinolyloxy)-2-propanol.

7) 1,3-Bis-(3-carboxy-1,4-dihydro-1-méthyl-4-oxo-7-quinonyloxy)-2-propanol.

8) 1,3-Bis-(3-carboxy-1,4-dihydro-4-oxo-7-quinolyloxy)-2-propanol.

9) 1,3-Bis-(3-carboxy-1,4-dihydro-4-oxo-6-quinolyloxy)-2-propanol.

10) 1,3-Bis-(1-éthyl-3-carboxy-1,4-dihydro-4-oxo-6-quinolyloxy)-2-propanol.

11) 1,3-Bis-(1-éthyl-3-carboxy-1,4-dihydro-4-oxo-6-quinolyloxy)-2-propanol.

12) 2-Acétoxy-1,3-bis-(1-éthyl-3-carboxy-1,4-dihydro-4-oxo-7-quinolyloxy)-propane.

13) Sel disodique du 1,3-bis-(1-éthyl-3-carboxy-1,4-dihydro-4-oxo-7-quinolyloxy)-2-propanol.

14) Préparations pharmaceutiques caractérisées en ce qu'elles contiennent un dérivé d'acide quinolone-carboxylique selon l'une quelconque des revendications 1 à 13.

15) Procédé de préparation des dérivés d'acide quinolone-carboxylique selon la revendication 1, caractérisé en ce que, ou bien

a) on condense une diamine de formule générale II

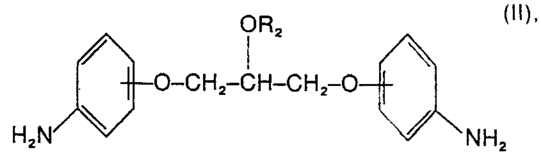

(II),

$R_2$ ayant la signification donnée à la revendication 1, avec un dérivé de l'acide malonique de formule III

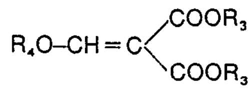

(III),

dans laquelle $R_3$ et $R_4$ représentent chacun un alkyle en $C_1$ à $C_6$, et on cyclise le composé de formule générale IV ainsi obtenu

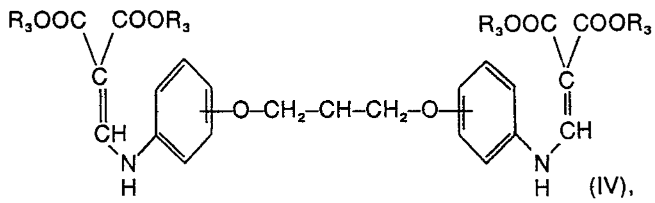

(IV),

ou bien,

b) on condense un dérivé d'acide hydroxy-quinolone-carboxylique de formule V

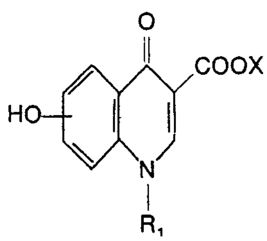

(V),

dans laquelle X et $R_1$ ont les significations indiquées à la revendication 1 et le groupe HO est à la position 6, 7 ou 8 du cycle de quinolone, avec un composé de formule générale VI ou VII

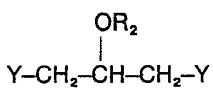

(VI),

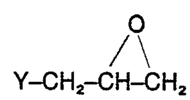

(VII),

$R_2$ ayant la signification donnée à la revendication 1 et Y représentant un atome de chlore, de brome ou d'iode ou un radical mésylate ou tosylate, et le cas échéant on saponifie ou on hydrolyse, on estérifie, on alkyle sur l'azote ou on transforme en un sel d'une base physiologiquement acceptable le composé obtenu suivant la variante a ou b.

**Revendication pour l'Etat contractant AT**

Procédé de préparation des dérivés d'acide quinolone-carboxylique de formule générale I

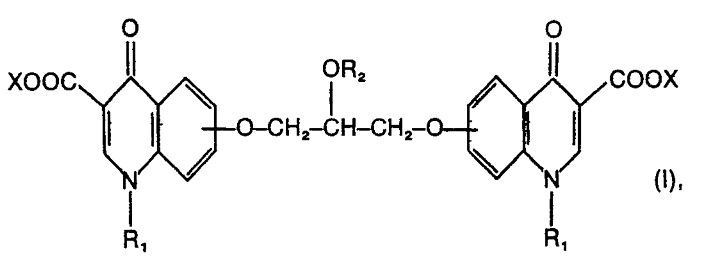

(I),

dans laquelle le groupement

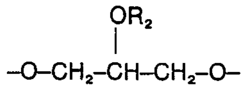

est à la position 6, 7 ou 8 de chacun des deux radicaux de quinolone et dans laquelle

$R_1$ représente un atome d'hydrogène ou un alkyle en $C_1$ à $C_6$,

$R_2$ un atome d'hydrogène ou un acyle en $C_1$ à $C_8$ et

X un atome d'hydrogène, le cation d'une base physiologiquement acceptable ou un alkyle en $C_1$ à $C_6$, procédé caractérisé en ce que ou bien

a) on condense une diamine de formule générale II

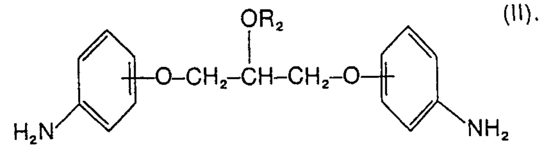

(II),

$R_2$ ayant la signification donnée à la revendication 1, avec un dérivé de l'acide malonique de formule III

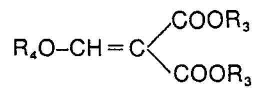

(III),

dans laquelle $R_3$ et $R_4$ représentent chacun un alkyle en $C_1$ à $C_6$, et on cyclise le composé de formule générale IV ainsi obtenu

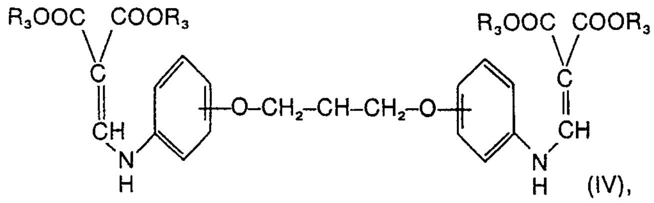

(IV),

ou bien,

b) on condense un dérivé d'acide hydroxy-quinolone-carboxylique de formule V

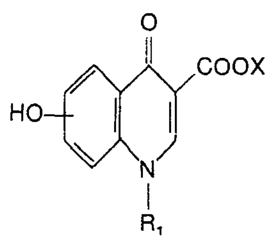

(V),

dans laquelle X et $R_1$ ont les significations indiquées à la revendication 1 et le groupe HO est à la position 6, 7 ou 8 du cycle de quinolone, avec un composé de formule générale VI ou VII

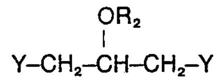

(VI),

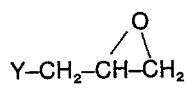

(VII),

$R_2$ ayant la signification donnée à la revendication 1 et Y représentant un atome de chlore, de brome ou d'iode ou un radical mésylate ou tosylate, et le cas échéant on saponifie ou on hydrolyse, on estérifie, on alkyle sur l'azote ou on transforme en un sel d'une base physiologiquement acceptable le composé obtenu suivant la variante a ou b.